# EUROPÄISCHE PATENTANMELDUNG

(11) **EP 4 327 817 A1**
(43) Veröffentlichungstag der Anmeldung: **28.02.2024**
(21) Anmeldenummer: 22191354.4
(22) Anmeldetag: 21.08.2022
(51) Int. Cl.: A61K 36/27, A61P 13/10, A61K 36/40, A61K 36/481, A61K 36/487, A61K 36/734, A61K 36/738, A61P 13/12

(54) **KRÄUTERMISCHUNG ZUR VERBESSERTEN NIEREN-UND BLASENFUNKTION UND GEGEN BLASENSCHWÄCHE**

(60) Teilanmeldung: 23197283.7
(71) Anmelder: Sun, Xiaochun, 12347 Berlin (DE)
(72) Erfinder: Sun, Xiaochun, 12347 Berlin (DE)

(57) **Zusammenfassung**

Die Erfindung betrifft eine Kräutermischung basierend auf TCM (Traditioneller Chinesischer Medizin), zur Verstärkung der Nieren- und Blasenfunktion gegen Blasenschwäche, sowie zur Leistungssteigerung des menschlichen Organismus. Die Erfindung unterstützt den Schliess- und Ringmuskel zu stärken für die verbesserte Kontolle beim Harngang.

Die Erfindung umfasst 6 Zutaten. Diese wird zu Granulat verarbeitet oder als Extrakt gewonnen und oral eingenommen.

Astragalus memeranaceus (Fisch.) 10-20%, Cynanchum otophyllum Schneid. 10-20%, Crataegus pinnatifida Bunge 10-20%, Psoralea corylifolia Linn., 20-30%, Rosa laevigata Michx. 20-30%, Cornus officinalis Sieb. et Zucc. 20-30%.

Diese Erfindung bezieht sich auf die Zusammensetzung und Extrakt des gleichen.

## Beschreibung

### Beschreibung der Ausführungsarten

Die Erfindung betrifft eine Kräutermischung basierend auf TCM (Traditioneller Chinesischer Medizin), zur Verstärkung der Nieren- und Blasenfunktion gegen Blasenschwäche, sowie zur Leistungssteigerung des menschlichen Organismus. Die Erfindung unterstützt den Schliess- und Ringmuskel zu stärken für die verbesserte Kontolle beim Harngang.

Die Kräuter Psoralea corylifolia Linn., Rosa laevigata Michx., Cornus officinalis Sieb. et Zucc. funktionieren in der TCM als Nierenstärkend und als Harnröhren Schrumpfmittel. Unterstützend wirken die Kräuter Astragalus memeranaceus (Fisch.), Cynanchum otophyllum Schneid., Crataegus pinnatifida Bunge.

Die Erfindung umfasst 6 Zutaten. Diese werden zu Granulat verarbeitet und oral eingenommen.

Astragalus memeranaceus (Fisch.) 10-20%, Cynanchum otophyllum Schneid. 10-20%, Crataegus pinnatifida Bunge 10-20%, Psoralea corylifolia Linn. 20-30%, Rosa laevigata Michx. 20-30%, Cornus officinalis Sieb. et Zucc. 20-30%. Die Zutaten werden zusammengemischt und zu Granulat verarbeitet und in Kapsel- oder Tablettenform oral eingenommen. Die Substanzen der Zutaten können mit traditioneller chinesischer Technik mit Alkohol zu Konzentrat extrahiert werden.

Die Kräutermischung kann auch in Wasser gekocht als Tee verzehrt werden.

## Patentansprüche

1. Zusammensetzung der Kräutschermischung mit tradionellen TCM Kräutern: Astragalus memeranaceus (Fisch.) 10-20%, Cynanchum otophyllum Schneid. 10-20%, Crataegus pinnatifida Bunge 10-20%, Psoralea corylifolia Linn., 20-30%, Rosa laevigata Michx. 20-30%, Cornus officinalis Sieb. et Zucc. 20-30%

2. Zusammensetzung gemäss Anspruch 1, mit Wirkung zur verbesserten Nieren- und Blasenfunktion und gegen Blasenschwäche

3. Zusammensetzung gemäss Anspruch 1, sowie dessen Extrakt
